# EUROPÄISCHE PATENTSCHRIFT

(11) **EP 3 688 459 B1**
(45) Veröffentlichungstag und Bekanntmachung des Hinweises auf die Patenterteilung: **10.03.2021**
(21) Anmeldenummer: 18781952.9
(22) Anmeldetag: 20.09.2018
(51) Int. Cl.: G01N 33/18, G01N 1/20

(54) **VORRICHTUNG ZUR ERFASSUNG DER QUALITÄT EINER FLÜSSIGKEIT IN EINEM VERSORGUNGSROHR**
DEVICE FOR DETECTING THE QUALITY OF A LIQUID IN A SUPPLY PIPE
DISPOSITIF DE DÉTECTION DE LA QUALITÉ D'UN LIQUIDE DANS UN TUBE D'ALIMENTATION

(30) Priorität: 25.09.2017 AT 508142017
(43) Veröffentlichungstag der Anmeldung: 05.08.2020
(73) Patentinhaber: s::can GmbH, 1200 Wien (AT)
(72) Erfinder: WEINGARTNER, Andreas, 2100 Korneuburg (AT); LINDNER, Michael, 1200 Wien (AT); MORAWEK, Roman, 1020 Wien (AT); SPIGAHT, Bernd, 79539 Lörrach (DE)
(74) Vertreter: Sonn & Partner Patentanwälte
(86) Internationale Anmeldenummer: PCT/AT2018/060219
(87) Internationale Veröffentlichungsnummer: WO 2019/056036

(56) Entgegenhaltungen:
- EP-A1- 2 345 886
- WO-A1-2013/172931
- CH-A2- 699 850
- FR-A1- 2 708 347
- US-A- 5 834 657
- US-A1- 2005 223 829

## Beschreibung

Die Erfindung betrifft eine Vorrichtung zur Erfassung der Qualität einer Flüssigkeit in einem Versorgungsrohr, insbesondere zur Erfassung der Wasserqualität in einem Wasserrohr, mit einer Durchflusszelle, welche eine Einlassöffnung, eine Auslassöffnung und zumindest eine Aufnahmevorrichtung zur Anordnung zumindest eines Sensors aufweist.

Die Erfindung betrifft zudem eine Kombination eines Versorgungsrohrs, insbesondere Wasserrohrs, mit der oben genannten Vorrichtung.

Schließlich betrifft die Erfindung ein Verfahren zum Verbinden der oben genannten Vorrichtung mit einem Versorgungsrohr, insbesondere einem Wasserrohr.

Vorrichtungen mit zumindest einem Sensor zur Überwachung der Qualität von Wasser sind bekannt.

Die Erfassung der Qualität von Flüssigkeiten erfolgt häufig durch Entnahme von Proben aus der Flüssigkeit und die externe Analyse der Proben mit Hilfe entsprechender Messgeräte. Nach der Analyse werden die entnommenen Flüssigkeitsproben entsorgt.

An manchen Stellen von Versorgungsrohren ist kein Kanalanschluss vorgesehen, weshalb die Entsorgung der entnommenen Flüssigkeitsproben nicht oder nicht einfach möglich ist. Daher wird die Erfassung der Qualität von Flüssigkeiten auch bevorzugt im Durchflussverfahren durchgeführt.

Beispielsweise offenbart die CH 699 850 A2 eine Sensoranordnung und ein Verfahren zur Überwachung der Wasserqualität in einer Wasserversorgungsleitung. Hierfür weist die Sensoranordnung eine Durchflusszelle mit mehreren Sensoren auf, die der Erfassung verschiedener Wasserqualitätsmerkmale und der Lieferung aktueller Messdaten dienen. Die Durchflusszelle weist einen Wassereinlass und einen Wasserauslass auf, die mit einem Zuflussrohr bzw. einem Abflussrohr der Wasserversorgungsleitung verbunden werden. Zwischen dem Wassereinlass und dem Wasserauslass erstreckt sich in der Sensoranordnung ein Einweg-Wasserströmungsweg, mit welchem die Sensoren in Eingriff stehen. Die Sensoranordnung umfasst auch eine Kommunikationseinheit zum Senden der aktuellen Messdaten an einen zentralen Regler.

Die derart ausgebildete Sensoranordnung lässt sich nur umständlich bzw. zeitaufwändig an der Wasserversorgungsleitung montieren und erschwert zudem die Durchführung von im Allgemeinen regelmäßig durchzuführenden Wartungsaufgaben am Versorgungsrohr.

Die EP 2 345 886 A1 offenbart eine Prüfzelle zur visuellen Prüfung eines in einer Leitung strömenden Fluids. Hierzu weist die Prüfzelle einen Prüfraum auf, der über einen Anschluss mit der Leitung verbunden, beispielsweise verschraubt wird. Durch diesen Anschluss wird Fluid aus der Leitung abgezweigt und in den Prüfraum geleitet. Der Prüfraum weist ein Sichtfenster in einer Außenwand auf, durch welches das abgezweigte Fluid beobachtet werden kann. Ein innerhalb des Anschlusses verlaufendes Innenrohr, welches sich bis in die Leitung hinein erstreckt, ist mit einer Eintrittsöffnung des Prüfraums verbunden, um das Fluid anzusaugen. Ein das Innenrohr umgebender Freiraum und/oder ein zweiter Kanal in dem Innenrohr und/oder ein weiteres Innenrohr dient als Rückfluss vom Prüfraum in die Leitung.

Die WO 2013/172931 A1 betrifft ein System und ein Verfahren zur Entnahme von Ballastwasser aus Schiffen, um dieses in unabhängigen Einrichtungen überprüfen zu lassen.

Die US 2005/0223829 A1 offenbart eine Vorrichtung zum Eintauchen einer Sonde oder ähnlichem in ein Versorgungsrohr mit einem unter Druck stehenden Fluid und zum Zurückziehen der Sonde. Die Sonde ist in der Höhe verstellbar in einem Innenrohr und dieses in einem Gehäuse aufgenommen.

Die Aufgabe der vorliegenden Erfindung besteht in der Schaffung einer Vorrichtung und eines Verfahrens der eingangs genannten Art, welche die Nachteile des Standes der Technik vermeiden oder zumindest verringern. Die Vorrichtung soll platzsparend und kostengünstig ausgebildet sein und sich auch nachträglich auf einfache Weise an einem Versorgungsrohr, insbesondere an einer Wasserversorgungsleitung, montieren lassen. Zudem soll die Vorrichtung die Wartung und den Austausch von damit verbundenen Sensoren erleichtern und auch regelmäßige Wartungsarbeiten am Versorgungsrohr zulassen.

Die erfindungsgemäßen Aufgaben werden durch eine oben genannte Vorrichtung gelöst, bei der die Einlassöffnung und die Auslassöffnung an einer zur Verbindung mit dem Versorgungsrohr vorgesehenen Basisfläche der Durchflusszelle vorgesehen sind, ein Ansaugrohr vorgesehen ist, die Einlassöffnung der Durchflusszelle mit dem Ansaugrohr verbunden ist, dessen freies Ende zur Anordnung im Versorgungsrohr vorgesehen ist, welches Ansaugrohr in seiner Längsrichtung verschiebbar in der Durchflusszelle aufgenommen oder in seiner Länge verstellbar ausgebildet ist, und eine Flüssigkeitspumpe, zum Pumpen eines aus der Flüssigkeit im Versorgungsrohr abgezweigten Stroms durch das Ansaugrohr, die Einlassöffnung, die Durchflusszelle und durch die Auslassöffnung, vorgesehen und mit dem Ansaugrohr verbunden ist und zumindest ein Sensor in der Durchflusszelle angeordnet ist und durch eine Spektrometersonde, eine ionenselektive Sonde, einen elektrochemischen Sensor oder einen optischen Sensor gebildet ist. Die Vorrichtung zur Erfassung der Qualität einer Flüssigkeit in einem Versorgungsrohr, insbesondere zur Erfassung der Wasserqualität in einem Wasserrohr mittels zumindest eines geeigneten Sensors, weist demnach eine Durchflusszelle auf, welche mit dem Sensor bzw. den Sensoren verbindbar ist. Für die Verbindung des zumindest einen Sensors mit der Durchflusszelle weist die Durchflusszelle zumindest eine Aufnahmevorrichtung auf. Vorzugsweise ist für jeden mit der Durchflusszelle zu verbindenden Sensor eine Aufnahmevorrichtung zur Anordnung bzw. zur Aufnahme des Sensors vorgesehen. Die Durchflusszelle weist zudem eine Einlassöffnung und eine Auslassöffnung auf, die an einer zur Verbindung mit dem Versorgungsrohr vorgesehenen Basisfläche der Durchflusszelle vorgesehen sind. Die Basisfläche der Durchflusszelle ist somit in einem Betriebszustand der Vorrichtung, in welchem die Durchflusszelle am Versorgungsrohr angeordnet ist, dem Versorgungsrohr zugewandt. Durch die Einlassöffnung kann dem Versorgungsrohr entnommene Flüssigkeit, deren Qualität zu erfassen ist, in die Durchflusszelle eingebracht werden. Nachdem die Flüssigkeit die Durchflusszelle durchlaufen hat und dabei mit den Sensoren in Kontakt getreten ist, kann sie durch die Auslassöffnung aus der Durchflusszelle wieder austreten und in das Versorgungsrohr zurück fließen. Hierfür sind die Einlassöffnung und die Auslassöffnung im Betriebszustand der Vorrichtung in Deckung mit einer Durchgangsbohrung in einer Wand des Versorgungsrohrs angeordnet. Die Strömung der zu analysierenden Flüssigkeit durch die Durchflusszelle wird mit einer Flüssigkeitspumpe erzielt bzw. aufrechterhalten. Die Flüssigkeitspumpe hält zweckmäßiger Weise einen im Wesentlichen konstanten Durchfluss von Flüssigkeit in der Durchflusszelle aufrecht, was sich günstig auf die Genauigkeit auswirkt, mit welcher die Qualität der Flüssigkeit erfasst wird. Dies kann damit begründet werden, dass die Messgenauigkeit mancher Sensoren von der Fließgeschwindigkeit der mit dem Sensor in Kontakt stehenden Flüssigkeit abhängt. Im Vergleich hierzu sind bei anderen Vorrichtungen ohne eine Flüssigkeitspumpe, bei welchen sich der Flüssigkeitsstrom durch die Durchflusszelle selbstständig einstellt, zusätzliche Maßnahmen zu ergreifen, um den Flüssigkeitsstrom konstant zu halten oder die Messgenauigkeit ist im Vergleich zur erfindungsgemäßen Vorrichtung geringer. Die Flüssigkeitspumpe trägt somit zu hoher Messgenauigkeit bei. Um die Durchflusszelle mit geringen Abmessungen, insbesondere mit einer kleinen Basisfläche herstellen zu können, ist es günstig, wenn die Einlassöffnung und die Auslassöffnung mit möglichst geringem Abstand nebeneinander angeordnet sind. Wegen des vorzugsweise geringen Abstands zwischen der Einlassöffnung und der Auslassöffnung würde ohne weitere Maßnahmen zumindest ein Teil der aus der Auslassöffnung austretenden Flüssigkeit wieder durch die Einlassöffnung in die Durchflusszelle gesaugt werden. Dies trifft insbesondere zu, wenn die Durchflusszelle mittels eines rohrförmigen Verbindungsstücks am Versorgungsrohr montiert und demnach in einem Abstand zum Versorgungsrohr angeordnet ist, da innerhalb des Verbindungsstücks nahezu keine durch die Flüssigkeit im Versorgungsrohr bedingte Flüssigkeitsströmung auftritt. Demnach würde ein Teil der bereits analysierten Flüssigkeit erneut gemessen. Um dies zu verhindern, d.h. um stets neue Flüssigkeit aus dem Versorgungsrohr in die Durchflusszelle zu leiten, ist die Einlassöffnung der Durchflusszelle mit einem Ansaugrohr verbunden. Das Ansaugrohr ist vorgesehen, mit seinem freien, der Durchflusszelle gegenüberliegenden Ende im Versorgungsrohr angeordnet, d.h. in das Versorgungsrohr eingesetzt zu werden. Demnach erstreckt sich das Ansaugrohr von der Durchflusszelle bzw. von ihrer Basisfläche in Richtung zum Versorgungsrohr, wenn an diesem die Durchflusszelle angeordnet ist. Mittels des Ansaugrohrs wird daher der Abstand zwischen jener Position, an welcher die Flüssigkeit aus dem Versorgungsrohr entnommen wird, und der Auslassöffnung vergrößert und eine Zirkulation von aus der Durchflusszelle ausgegebener Flüssigkeit erneut in die Durchflusszelle hinein vermieden. Um eine geeignete Positionierung des freien Endes des Ansaugrohrs zu gewährleisten, ist das Ansaugrohr in seiner Längsrichtung verschiebbar bzw. verstellbar in der Durchflusszelle aufgenommen oder in seiner Länge verstellbar ausgebildet. Somit kann das freie Ende des Ansaugrohrs in Bezug auf die Durchflusszelle, insbesondere in Bezug auf die Basisfläche der Durchflusszelle, verschoben werden. Das Ansaugrohr ist zweckmäßiger Weise flüssigkeitsdicht mit der Einlassöffnung verbunden und es ist jedenfalls mit der Flüssigkeitspumpe verbunden, um Flüssigkeit aus dem Versorgungsrohr durch das Ansaugrohr und durch die Durchflusszelle leiten zu können. Wenn das Ansaugrohr in seiner Länge verstellbar ausgebildet ist, kann es beispielsweise zumindest zwei teleskopartig gegeneinander verschiebbare Teile aufweisen. Mit der beschriebenen Vorrichtung kann die Qualität der Flüssigkeit in situ erfasst werden, ohne dass Proben entnommen und nach der Analyse entsorgt werden müssen. Somit kann die Vorrichtung auch an Stellen des Versorgungsrohrs installiert werden, wo kein Abfluss oder Kanal vorgesehen ist. Zudem kann die Vorrichtung wegen der kleinen Basisfläche platzsparend ausgebildet sein. Weiters kann die Vorrichtung kostengünstig hergestellt werden, da das Ansaugrohr und die Flüssigkeitspumpe selbst nur geringe Kosten verursachen.

Wenn im Rahmen der Beschreibung auf die Positionsangaben oben oder unten an der Durchflusszelle Bezug genommen wird, so ist dies unter der Annahme zu verstehen, dass sich die Basisfläche unten an der Durchflusszelle befindet. Hierdurch wird nicht ausgeschlossen, dass die Durchflusszelle betriebsbereit oberhalb oder seitlich des Versorgungsrohrs mit dem Versorgungsrohr verbunden ist.

Im Gegensatz zu bekannten Vorrichtungen, die ein erstes Rohr zur Verbindung mit einer Einlassöffnung der Durchflusszelle und ein zweites Rohr zur Verbindung mit einer Auslassöffnung der Durchflusszelle vorsehen, weist die erfindungsgemäße Vorrichtung eine einzige Anschlussstelle zur Verbindung der Durchflusszelle mit dem Versorgungsrohr auf. Für die Herstellung einer Verbindung der Durchflusszelle mit dem Versorgungsrohr ist daher im Gegensatz zur bekannten Vorrichtung nur eine einzige Öffnung in das Versorgungsrohr einzubringen. Die hierdurch erzielt Zeiteinsparung für die Montage der Vorrichtung am Versorgungsrohr führt zu einer Reduktion des Aufwands und der Kosten. Zudem führt die Vorrichtung mit einer einzigen Anschlussstelle zu einer Einsparung von Materialkosten. Da zur Verbindung mit der Vorrichtung nur eine einzige Öffnung in das Versorgungsrohr einzubringen ist, unterliegt der Montageort der Vorrichtung geringeren Einschränkungen. Zudem belegt die Vorrichtung insgesamt einen kürzeren Längsabschnitt am Versorgungsrohr, wodurch die Montagekosten weiter reduziert werden können. Besonders günstig ist, dass die Vorrichtung rasch und einfach, nachträglich an einem bestehenden, unter Druck stehenden Versorgungsrohr montiert werden kann.

Gemäß einer bevorzugten Ausführungsform der Erfindung kann vorgesehen sein, dass sich in einem mit dem Versorgungsrohr verbundenen Betriebszustand der Durchflusszelle das freie Ende des Ansaugrohrs bis zu einer Wand des Versorgungsrohrs erstreckt. Das Ansaugrohr ist somit ausgebildet und angeordnet, sich im Betriebszustand der Durchflusszelle mit seinem freien Ende bis zu einer Wand des Versorgungsrohrs, insbesondere bis zu einer Innenfläche der Wand des Versorgungsrohrs, zu erstrecken. Damit wird gewährleistet, dass direkt aus dem Versorgungsrohr entnommene Flüssigkeit, die höchstens in geringem Ausmaß Anteile der von der Durchflusszelle ausgegebenen Flüssigkeit aufweist, durch das Ansaugrohr und durch die Durchflusszelle gepumpt wird. Wenn sich das Ansaugrohr nicht in das Innere des Versorgungsrohrs, d.h. über die Innenwand des Versorgungsrohrs hinaus erstreckt, können jederzeit Wartungsarbeiten im Versorgungsrohr durchgeführt werden, ohne die Position des Ansaugrohrs verändern zu müssen.

Für eine kompakte Ausführung der Durchflusszelle ist es günstig, wenn die Flüssigkeitspumpe an der Durchflusszelle angebracht ist. Beispielsweise kann die Flüssigkeitspumpe lösbar an der Basisfläche der Durchflusszelle montiert sein. Auf diese Weise kann die Flüssigkeitspumpe bei Bedarf, etwa um ausgetauscht zu werden, von der Durchflusszelle abgenommen werden. Da für die Förderung der Flüssigkeit durch die Durchflusszelle nur geringe Energie erforderlich ist, kann die Flüssigkeitspumpe für eine Leistung von etwa 1 W ausgelegt und beispielsweise batteriebetrieben sein.

Zur Vermeidung eines Austritts von Flüssigkeit aus dem Versorgungsrohr oder aus der Durchflusszelle, kann zumindest eine Verschlusseinrichtung zum Absperren des Stroms der Flüssigkeit im Versorgungsrohr in Richtung zur Durchflusszelle bzw. von der Durchflusszelle weg vorgesehen sein. Mittels der Verschlusseinrichtung kann daher der aus dem Versorgungsrohr abgezweigte und durch die Durchflusszelle strömende Flüssigkeitsstrom angehalten werden, ohne das Versorgungsrohr absperren zu müssen. Die Verschlusseinrichtung kann in der Durchflusszelle selbst oder besonders bevorzugt am Versorgungsrohr vorgesehen sein. Wenn die Verschlusseinrichtung am Versorgungsrohr angeordnet ist, um bedarfsweise die Öffnung im Versorgungsrohr abzudichten, welche zum Einsetzen des Ansaugrohrs vorgesehen ist, kann die gesamte erfindungsgemäße Vorrichtung von dem Versorgungsrohr abgenommen werden, ohne das Versorgungsrohr absperren zu müssen. Die Verschlusseinrichtung ermöglicht somit, dass nach dem Verschließen der Verschlusseinrichtung die mit dem abgezweigten Flüssigkeitsstrom in Verbindung stehenden Sensoren aus der Durchflusszelle entnommen werden können, ohne einem unerwünschten nennenswerten Flüssigkeitsaustritt aus der Durchflusszelle. Die Verschlusseinrichtung kann beispielsweise durch einen zwischen zwei Dichtungen aufgenommenen, manuell zu betätigenden, insbesondere quer zur Strömungsrichtung verschiebbaren Schiebekörper gebildet sein.

Besonders günstig ist es, wenn eine Verstelleinrichtung für das Ansaugrohr zum Verstellen des Ansaugrohrs in Längsrichtung des Ansaugrohrs zwischen einer eingezogenen, im Betriebszustand der Durchflusszelle vom Versorgungsrohr entfernten Position und einer ausgefahrenen, im Betriebszustand der Durchflusszelle zum Versorgungsrohr erstreckten Position, vorgesehen ist. Die Verstelleinrichtung erleichtert die Montage der Vorrichtung am Versorgungsrohr, da hiermit auch nach der Herstellung einer Verbindung der Vorrichtung mit dem Versorgungsrohr der Abstand des freien Endes des Ansaugrohrs von der Basisfläche der Durchflusszelle verändert werden kann. Insbesondere kann das freie Ende des Ansaugrohrs bis zur Innenwand des Versorgungsrohrs ausgefahren werden. Es ist jedoch nicht ausgeschlossen, dass das freie Ende des Ansaugrohrs bis in das Versorgungsrohr hineinreichend ausgefahren werden kann. Für die Verstellung des Ansaugrohrs kann dieses beispielsweise mittels einer damit verbundenen Betätigungsstange, welche zur Betätigung durch einen Benutzer aus der Durchflusszelle herausragt, entlang seiner Längserstreckung aus dem Versorgungsrohr herausgezogen oder in dieses eingeschoben werden. Gemäß einem anderen Beispiel kann das Ansaugrohr entlang seiner Längserstreckung einen Gewindeabschnitt aufweisen, welcher in einen korrespondierenden Gewindeabschnitt in der Durchflusszelle oder in einem mit der Durchflusszelle verbundenen Stützkörper eingreift. Für die Verstellung des Ansaugrohrs ist günstiger Weise ein Handgriff vorgesehen. Besonders vorteilhaft ist es, wenn das Ansaugrohr verschiebbar in einem Stützkörper aufgenommen ist, welcher zur lösbaren Anordnung in der Durchflusszelle eingerichtet ist.

Um Verunreinigungen der Durchflusszelle bzw. Sensoren zu vermeiden, kann in der Durchflusszelle, in Strömungsrichtung der Flüssigkeit vor der zumindest einen Aufnahmevorrichtung, oder im Ansaugrohr ein Sedimentfilter vorgesehen sein. Dadurch können auch die Intervalle zur Wartung der Durchflusszelle erhöht werden. Zweckmäßiger Weise ist das Sedimentfilter lösbar befestigt, um es bei Bedarf reinigen oder austauschen zu können.

Günstig ist, wenn die Flüssigkeitspumpe zum Pumpen der Flüssigkeit in entgegengesetzte Richtungen ausgebildet ist oder eine Umleiteinrichtung zum Umkehren der Strömungsrichtung der Flüssigkeit durch das Sedimentfilter vorgesehen ist. Demnach kann die Flüssigkeitspumpe zur Umkehrung der Drehrichtung umschaltbar ausgebildet sein. Alternativ zu einer Flüssigkeitspumpe, die zum Pumpen der Flüssigkeit in entgegengesetzte Richtungen ausgebildet ist, kann die Umleiteinrichtung vorgesehen sein. Vorzugsweise weist die Umleiteinrichtung Rohrleitungen und ein Wegeventil zum Umleiten der Flüssigkeit durch die Rohrleitungen auf. Somit kann das Sedimentfilter durch Umkehren der Strömungsrichtung der Flüssigkeit durch das Sedimentfilter gereinigt werden.

Gemäß einer weiteren Ausführungsform kann zumindest ein Sensor in der Durchflusszelle durch einen Durchflusssensor oder einen Drucksensor gebildet sein. Selbstverständlich können mehrere Sensoren, insbesondere eine Kombination der vorstehend genannten Sensoren, in der Durchflusszelle angeordnet sein. Insbesondere kann zumindest ein Sensor in der Durchflusszelle angeordnet sein, der die Leitfähigkeit der Flüssigkeit bzw. von Wasser, einen Anteil von freiem Chlor, einen Gehalt von Gesamtchlor, einen pH-Wert, eine Trübung der Flüssigkeit, einen TOC (Total Organic Carbon)-Wert und/oder die Farbe der Flüssigkeit bzw. des Wassers erfasst.

Wenn zumindest eine Aufnahmevorrichtung zum Einschrauben oder Einstecken zumindest eines Sensors in die Durchflusszelle ausgebildet ist, kann der Sensor auf einfache und rasche Weise zuverlässig in der Aufnahmevorrichtung angeordnet und daraus wieder entnommen werden. Die hierfür vorgesehene Schraubverbindung oder Steckverbindung der Aufnahmevorrichtung und des Sensors ist vorzugsweise flüssigkeitsdicht ausgebildet. Insbesondere kann für jeden in der Durchflusszelle anzuordnenden Sensor jeweils eine Schraubverbindung oder Steckverbindung vorgesehen sein. Jene Aufnahmevorrichtungen, in welchen unabhängig von der Art der Verbindung mit dem Sensor kein Sensor angeordnet ist, werden bevorzugt mit einem Verschlusselement flüssigkeitsdicht verschlossen. Im Falle der Schraubverbindung/Steckverbindung kann das Verschlusselement ein mit/in der Aufnahmevorrichtung verschraubbarer/einsteckbarer Deckel sein. Zudem kann eine Aufnahmevorrichtung zur Anordnung, insbesondere zum Einschrauben oder Einstecken, des Ansaugrohrs oder eines das Ansaugrohr umgebenden Stützkörpers in die Durchflusszelle vorgesehen sein.

Für eine stabile Ausbildung der erfindungsgemäßen Vorrichtung können die Durchflusszelle und/oder das Ansaugrohr zumindest teilweise aus Metall und/oder Kunststoff, vorzugsweise Polyoxymethylen, gebildet sein. Insbesondere kann die Durchflusszelle in einem unteren, an die Basisfläche anschließenden Bereich, aus Edelstahl und in einem oberen, die Aufnahmevorrichtung aufweisenden Bereich, aus Trinkwasser-zugelassenem Polyoxymethylen gebildet sein. Aus Gründen der Hygiene wird ein Ansaugrohr aus Edelstahl bevorzugt.

Um die Erfassung der Qualität der Flüssigkeit im Versorgungsrohr durch angesaugte Luft aus dem Versorgungsrohr nicht zu beeinträchtigen, kann die Durchflusszelle eine Entlüftungsvorrichtung, insbesondere ein Entlüftungsventil, aufweisen oder damit verbunden sein. Günstiger Weise ist die Entlüftungsvorrichtung zur automatischen Entlüftung ausgebildet und in einem oberen Bereich der Durchflusszelle angeordnet.

Wenn die Durchflusszelle eine Entnahmevorrichtung, vorzugsweise einen Kugelhahn, zur Entnahme einer Flüssigkeitsprobe aufweist, kann die Qualität der Flüssigkeitsprobe in einer zur erfindungsgemäßen Vorrichtung externen Einrichtung erfasst werden. Die Entnahmevorrichtung ermöglicht somit einen einfachen Zugang zur Flüssigkeit im Versorgungsrohr und gegebenenfalls die Erfassung von Merkmalen der Flüssigkeit, welche mit den Sensoren in der Durchflusszelle nicht erfasst werden können. Die Entnahmevorrichtung kann zur manuellen oder automatischen Betätigung eingerichtet sein.

Die erfindungsgemäße Aufgabe wird auch durch eine Kombination eines Versorgungsrohrs, insbesondere Wasserrohrs, mit der zuvor beschriebenen Vorrichtung gelöst, wobei das Ansaugrohr der Vorrichtung durch eine einzige Öffnung in einer Wand des Versorgungsrohrs in das Versorgungsrohr ragt. Die Kombination kann zudem ein vorzugsweise rohrförmiges Verbindungsstück zwischen der Durchflusszelle und dem Versorgungsrohr aufweisen, mit welchem Verbindungsstück die Durchflusszelle mit dem Versorgungsrohr verbunden ist. Hinsichtlich der Vorteile, die sich aus der einzigen Öffnung im Versorgungsrohr ergeben, wird auf die obige Beschreibung verwiesen.

Die erfindungsgemäße Aufgabe wird auch durch ein genanntes Verfahren gelöst, bei dem eine Durchgangsbohrung in eine Wand des Versorgungsrohrs eingebracht wird und an der Wand, mit der Durchgangsbohrung fluchtend, eine Verschlusseinrichtung zum Absperren des Stroms der Flüssigkeit im Versorgungsrohr in Richtung zur Durchflusszelle bzw. von der Durchflusszelle weg, und darauf folgend die Durchflusszelle fixiert werden. Insbesondere werden das freie Ende des Ansaugrohrs und die Auslassöffnung der Durchflusszelle mit der Durchgangsbohrung fluchtend angeordnet. Die mit der Durchgangsbohrung fluchtende Anordnung der Durchflusszelle gewährleistet die für den Betrieb der Vorrichtung nötige Flüssigkeitsverbindung zwischen dem Versorgungsrohr, dem freien Ende des Ansaugrohrs und der Auslassöffnung der Durchflusszelle. Zu den mit diesem Verfahren erzielbaren Vorteilen wird auch auf die obige Beschreibung der Vorrichtung verwiesen. Das Verfahren zum Verbinden der zuvor beschriebenen Vorrichtung mit einem Versorgungsrohr, insbesondere einem Wasserrohr, kann besonders einfach durchgeführt werden, da nur eine einzige Bohrung in die Wand des Versorgungsrohrs eingebracht werden muss.

Gemäß einer bevorzugten Ausführungsform des Verfahrens kann vorzugsweise nach dem Fixieren der Durchflusszelle am Versorgungsrohr das Ansaugrohr mittels einer Verstelleinrichtung für das Ansaugrohr bis zum Versorgungsrohr aus der Durchflusszelle ausgefahren werden, welche Verstelleinrichtung zum Verstellen des Ansaugrohrs in Längsrichtung des Ansaugrohrs zwischen einer eingezogenen, im Betriebszustand der Durchflusszelle vom Versorgungsrohr entfernten Position und einer ausgefahrenen, im Betriebszustand der Durchflusszelle zum Versorgungsrohr erstreckten Position, vorgesehen ist. Wenn die Verstelleinrichtung für das Ansaugrohr vorgesehen ist, kann die Position des freien Endes des Ansaugrohrs in Bezug auf das Versorgungsrohr nach dem Fixieren der Durchflusszelle am Versorgungsrohr eingestellt werden. Falls keine Verstelleinrichtung vorhanden ist, kann die Position des freien Endes des Ansaugrohrs durch die geeignete Wahl der Länge des Ansaugrohrs bestimmt werden.

Wenn zumindest eine Verschlusseinrichtung zum Absperren des aus dem Versorgungsrohr abgezweigten Flüssigkeitsstroms in Richtung zur Durchflusszelle bzw. von der Durchflusszelle weg vorgesehen ist, kann vor dem Ausfahren des Ansaugrohrs aus der am Versorgungsrohr fixierten Durchflusszelle die Verschlusseinrichtung geöffnet werden. Auf diese Weise wird durch Schließen der Verschlusseinrichtung verhindert, dass vor der Verbindung der Durchflusszelle mit dem Versorgungsrohr oder vor der Verbindung des Sensors mit der Aufnahmevorrichtung Flüssigkeit unerwünschter Weise aus den Verbindungsstellen austritt. Wenn die Verbindungen der Durchflusszelle mit dem Versorgungsrohr und die Verbindung des Sensors mit der Aufnahmevorrichtung hergestellt sind, wird durch Öffnen der Verschlusseinrichtung der Strömungsweg für die aus dem Versorgungsrohr abgezweigte, zu analysierende Flüssigkeit durch die Durchflusszelle freigegeben.

Die Erfindung wird im Folgenden anhand von bevorzugten, nicht einschränkenden Ausführungsbeispielen unter Bezugnahme auf die Zeichnungen noch weiter erläutert. Es zeigen:
Fig. 1 eine Vorrichtung gemäß der Erfindung mit einer Durchflusszelle und einem Ansaugrohr, in einer perspektivischen Ansicht;
Fig. 2a das Ansaugrohr der Vorrichtung aus Fig. 1 in einem eingezogenen Zustand in einer perspektivischen Ansicht;
Fig. 2b das Ansaugrohr der Vorrichtung aus Fig. 1 in einem ausgefahren Zustand in einer perspektivischen Ansicht;
Fig. 2c das Ansaugrohr der Vorrichtung aus Fig. 1 in einem ausgefahren Zustand in einer Schnittansicht;
Fig. 3a die Vorrichtung aus Fig. 1 in einem mit einem Versorgungsrohr verbundenen Zustand, in einer perspektivischen Ansicht;
Fig. 3b die Vorrichtung aus Fig. 1 in einem mit einem Versorgungsrohr verbundenen Zustand, in einer perspektivischen Ansicht aus einer zu Fig. 3a entgegengesetzten Richtung;
Fig. 4a die Vorrichtung aus Fig. 1 in einem mit einem Versorgungsrohr verbundenen Zustand, mit eingezogenem Ansaugrohr und mit einer verschlossenen Verschlusseinrichtung, in einer Schnittansicht quer zur Längsrichtung des Versorgungsrohrs;
Fig. 4b die Vorrichtung aus Fig. 1 in einem mit einem Versorgungsrohr verbundenen Zustand, mit ausgefahrenem Ansaugrohr und mit einer geöffneten Verschlusseinrichtung, in einer Schnittansicht quer zur Längsrichtung des Versorgungsrohrs; und
Fig. 5 eine schematisch dargestellte Umleiteinrichtung zum Umkehren der Strömungsrichtung der Flüssigkeit durch ein Sedimentfilter in der Vorrichtung aus Fig. 1.

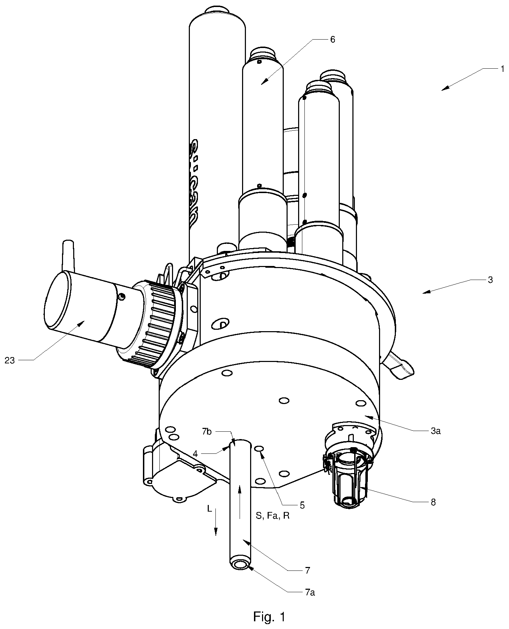

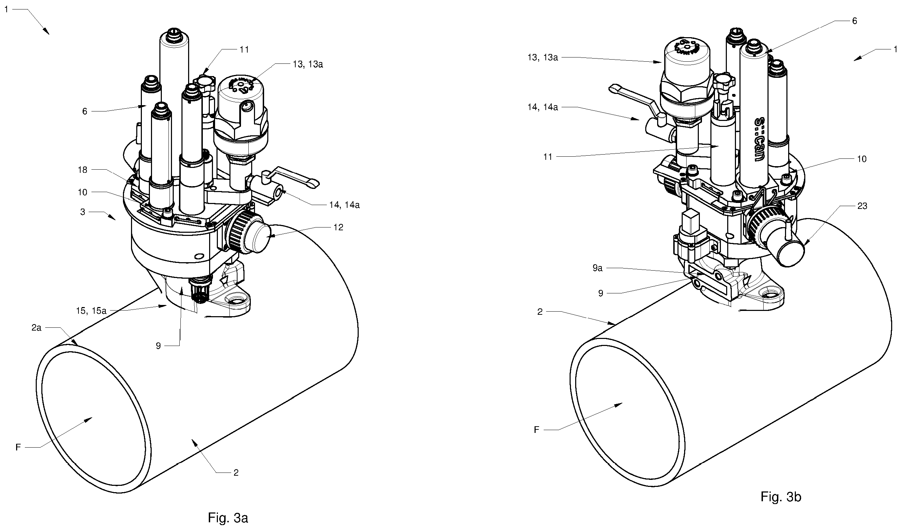

Fig. 1 zeigt die Vorrichtung 1 zur Erfassung der Qualität einer Flüssigkeit F in einem Versorgungsrohr 2 (in Fig. 1 nicht dargestellt), welche eine Durchflusszelle 3 und ein damit verbundenes oder verbindbares Ansaugrohr 7 aufweist. An einer Basisfläche 3a der Durchflusszelle 3, welche Basisfläche 3a dem Versorgungsrohr 2 zugewandt ist, wenn die Durchflusszelle 3 mit dem Versorgungsrohr 2 im Betriebszustand der Vorrichtung 1 verbunden ist, weist die Durchflusszelle 3 eine Einlassöffnung 4 und eine Auslassöffnung 5 auf. Dabei sind die Einlassöffnung 4 und die Auslassöffnung 5 möglichst nahe aneinander angeordnet, um gemeinsam über einer einzigen Öffnung 15 im Versorgungsrohr 2 angeordnet werden zu können. Die Einlassöffnung 4 ist mit dem Ansaugrohr 7, insbesondere mit einem Ende 7b des Ansaugrohrs 7 verbunden, um Flüssigkeit F, insbesondere Wasser, aus dem Versorgungsrohr 2 durch das Ansaugrohr 7 und durch die Einlassöffnung 4 der Durchflusszelle 3 zuführen und durch die Auslassöffnung 5 wieder aus der Durchflusszelle 3 in das Versorgungsrohr 2 ableiten zu können. Die Durchflusszelle 3 weist zwischen der Einlassöffnung 4 und der Auslassöffnung 5 einen nicht dargestellten Flüssigkeitskanal auf, entlang welchem die abgezweigte Flüssigkeit Fa über darin angeordnete Sensoren 6 strömt. Zur Erfassung der Qualität der Flüssigkeit Fa (Fig. 3a, 3b), die mittels des Ansaugrohrs 7 aus dem Versorgungsrohr 2, insbesondere aus einem Wasserrohr, abgezweigt wird, weist die Durchflusszelle 3 zumindest eine Aufnahmevorrichtung 10 zur Anordnung zumindest eines Sensors 6 auf. Gemäß dem dargestellten Ausführungsbeispiel sind vier Sensoren 6 mit der Durchflusszelle 3 verbunden, wobei vorzugsweise jedem Sensor 6 einen eigene Aufnahmevorrichtung 10 zugeordnet ist. Die Aufnahmevorrichtung 10 kann ein Gewinde oder Stecksystem 18 (Fig. 3a) zum Einbringen, insbesondere Einschrauben oder Einstecken, des Sensors 6 in die Durchflusszelle 3 aufweisen. Der Sensor 6 weist hierfür ein korrespondierendes Gewinde oder Stecksystem auf. Selbstverständlich kann die Durchflusszelle 3 zur Aufnahme von beliebig vielen Sensoren 6 ausgebildet sein. Die Anzahl der mit der Durchflusszelle 3 verbundenen Sensoren 6 kann geringer als die Zahl der Aufnahmevorrichtungen 10 sein, wobei die dabei freien Aufnahmevorrichtungen 10 mit einem nicht dargestellten Verschluss flüssigkeitsdicht abgeschlossen werden können.

Fig. 1 zeigt zudem eine Flüssigkeitspumpe 8 zum Pumpen eines aus der Flüssigkeit F im Versorgungsrohr 2 abgezweigten Stroms S durch das Ansaugrohr 7, die Einlassöffnung 4, die Durchflusszelle 3 und durch die Auslassöffnung 5. Die Flüssigkeitspumpe 8 ist hierfür mit dem Ansaugrohr 7, der Einlassöffnung 4, dem nicht dargestellten Flüssigkeitskanal in der Durchflusszelle 3, in welchem die Sensoren 6 angeordnet sind, und der Auslassöffnung 5 verbunden. Die Flüssigkeitspumpe 8 ist im in Fig. 1 dargestellten Beispiel an der Basisfläche 3a der Durchflusszelle 3 angebracht. In Fig. 1 ist auch eine Reinigungsvorrichtung 23 zum Reinigen der in der Durchflusszelle 3 angeordneten Sensoren 6 erkennbar.

Wie den Fig. 3a und 3b besser zu entnehmen ist, kann auch ein Sedimentfilter 12 vorgesehen sein, welches in der Durchflusszelle 3, in Strömungsrichtung der abgezweigten Flüssigkeit Fa vor der zumindest einen Aufnahmevorrichtung 10 angeordnet sein kann. Alternativ kann das Sedimentfilter 12 im Ansaugrohr 7 angeordnet sein. Das Sedimentfilter 12 filtert Partikel aus der abgezweigten Flüssigkeit Fa, welche die Messung der Sensoren 6 verfälschen könnten oder die Wartungsintervalle der Sensoren 6 oder jene der Durchflusszelle 3 reduzieren würden.

In den Fig. 3a und 3b ist zudem eine Entlüftungsvorrichtung 13, welche insbesondere ein Entlüftungsventil 13a sein kann, erkennbar. Über die Entlüftungsvorrichtung 13 wird Luft, die mittels der Flüssigkeitspumpe 8 aus dem Versorgungsrohr 2 in die Durchflusszelle 3 eingesaugt wurde, aus der Durchflusszelle 3 in die Umgebung abgeleitet. Um Flüssigkeitsproben entnehmen zu können, kann die Durchflusszelle 3 eine Entnahmevorrichtung 14, beispielsweise einen manuell betätigbaren Kugelhahn 14a, aufweisen. Wie weiters in den Fig. 3a und 3b zu erkennen ist, kann in der Durchflusszelle 3 eine Verstelleinrichtung 11 für das Ansaugrohr 7 vorgesehen sein. Die Verstelleinrichtung 11 dient dem Verstellen des Ansaugrohrs 7 in seiner Längsrichtung L zwischen einer eingezogenen Position und einer ausgefahrenen Position. Die Verstelleinrichtung 11 kann so wie die Sensoren 6 lösbar in der Aufnahmevorrichtung 10 aufgenommen sein.

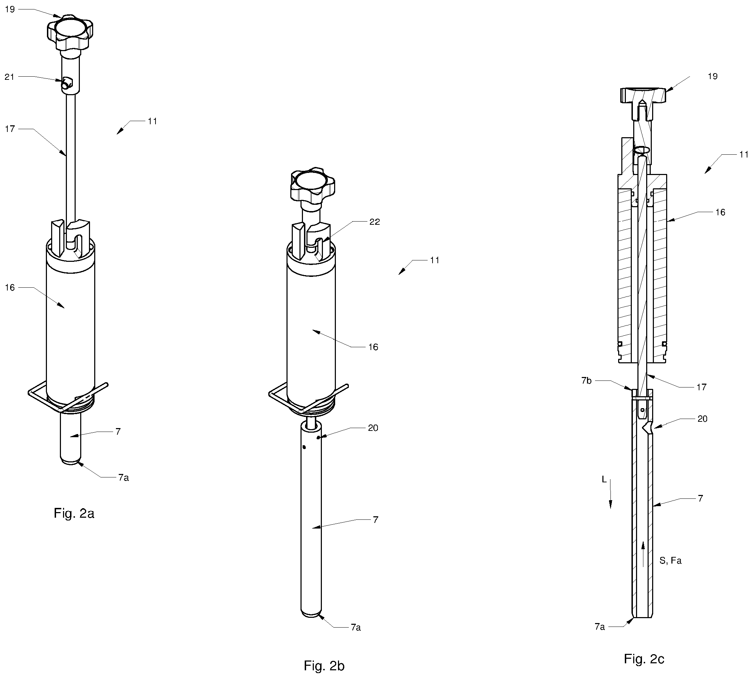

Die Fig. 2a bis 2c zeigen die Verstelleinrichtung 11 detaillierter. Die Verstelleinrichtung 11 kann ein Führungsrohr 16 aufweisen, in welchem eine Betätigungsstange 17 verschiebbar aufgenommen ist, welche mit dem Ansaugrohr 7 verbunden ist. Somit kann durch Verschieben der Betätigungsstange 17 in Längsrichtung L des Ansaugrohrs 7 die Position des Ansaugrohrs 7 verstellt werden. Dabei zeigt Fig. 2a das Ansaugrohr 7 in einer in das Führungsrohr 16 bzw. in die Durchflusszelle 3 eingezogenen Position, in welcher es im Betriebszustand der Durchflusszelle 3 vom Versorgungsrohr 2 entfernt ist. Demgegenüber zeigt Fig. 2b das Ansaugrohr 7 in einer aus dem Führungsrohr 16 bzw. aus der Durchflusszelle 3 ausgefahrenen Position, in welcher es im Betriebszustand der Durchflusszelle 3 zum Versorgungsrohr 2 erstreckt ist.

Zur Abgabe des Stroms S der abgezweigten Flüssigkeit Fa aus dem Ansaugrohr 7 in die Durchflusszelle 3, insbesondere in den nicht dargestellten Flüssigkeitskanal, entlang welchem der abgezweigte Flüssigkeitsstrom S über die darin angeordneten Sensoren 6 strömt, weist das Ansaugrohr 7 Öffnungen 20 auf. Im eingezogen Zustand des Ansaugrohrs 7 sind die Öffnungen 20 günstiger Weise durch die Innenwand des Führungsrohrs 16 abgedichtet. Die Betätigungsstange 17 ist günstiger Weise mit einem durch einen Benutzer einfach zu ergreifenden Handgriff 19 versehen. Zudem können an der Betätigungsstange 17 ein Sicherungsbolzen 21 und am Führungsrohr 16 ein Rastelement 22 zum gegenseitigen Einrasten vorgesehen sein. Beispielsweise kann der Sicherungsbolzen 21 durch Drehen des Handgriffs 19 mit dem Rastelement 22 in Eingriff gelangen, wodurch ein unbeabsichtigtes Herausziehen des Ansaugrohrs 7 aus dem Versorgungsrohr 2, d.h. ein Einziehen des Ansaugrohrs 7 in das Führungsrohr 16 bzw. in die Durchflusszelle 3, verhindert wird.

Die Fig. 3a und 3b zeigen eine Kombination des Versorgungsrohrs 2, insbesondere Wasserrohrs, mit der Vorrichtung 1. Insbesondere ist die Vorrichtung 1 in einem am Versorgungsrohr 2 montierten Zustand aus zwei entgegengesetzten Richtungen betrachtet dargestellt. Dabei ist erkennbar, dass zwischen der Vorrichtung 1, insbesondere der Durchflusszelle 3, und dem Versorgungsrohr 2 eine Verschlusseinrichtung 9 vorgesehen ist. Die Verschlusseinrichtung 9 dient dem Absperren des Stroms S der Flüssigkeit F im Versorgungsrohr 2 in Richtung zur Durchflusszelle 3 bzw. von der Durchflusszelle 3 weg. Hierfür kann beispielsweise ein in eine Öffnung 9a der Verschlusseinrichtung 9 einschiebbarer Plattenkörper die Flüssigkeitsverbindung zwischen dem Versorgungsrohr 2 und der Durchflusszelle 3 verschließen. Die im dargestellten Beispiel im Wesentlichen rohrförmig ausgebildete Verschlusseinrichtung 9 dient auch als Verbindungsstück zwischen der Durchflusszelle 3 und dem Versorgungsrohr 2.

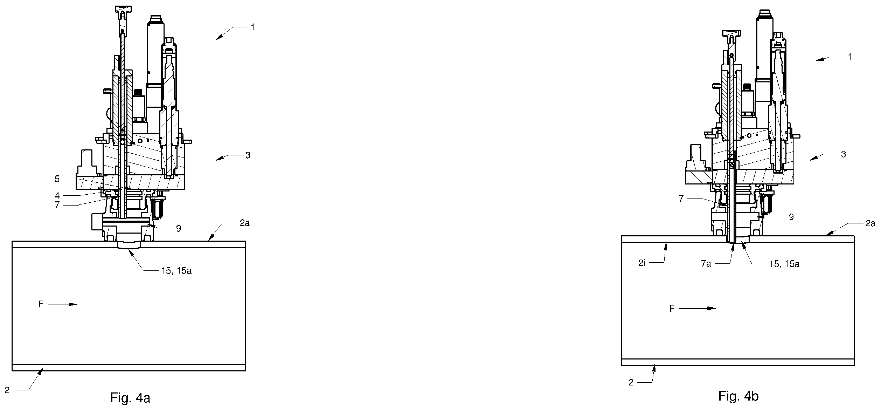

Die Fig. 4a und 4b zeigen die Kombination des Versorgungsrohrs 2, insbesondere Wasserrohrs, mit der Vorrichtung 1 in einer Schnittansicht. Es ist deutlich erkennbar, dass die Durchflusszelle 3, insbesondere die Einlassöffnung 4 und das Ansaugrohr 7, sowie die Auslassöffnung 5, über einer einzigen Durchgangsbohrung 15a in einer Wand 2a des Versorgungsrohrs 2 angeordnet sind.

In Fig. 4a ist die Vorrichtung 1 in einem mit dem Versorgungsrohr 2 verbundenen Betriebszustand gezeigt, wobei das Ansaugrohr 7 eingezogen und die Verschlusseinrichtung 9 verschlossen ist. Somit können beispielsweise die Sensoren 6 bei geschlossener Verschlusseinrichtung 9, ohne unerwünschten Flüssigkeitsaustritt aus der Durchflusszelle 3, entnommen oder ausgetauscht werden.

In Fig. 4b ist die Vorrichtung 1 in einem mit dem Versorgungsrohr 2 verbundenen Betriebszustand gezeigt, wobei das Ansaugrohr 7 ausgefahren und die Verschlusseinrichtung 9 geöffnet ist. Das der Durchflusszelle 3 gegenüberliegende untere bzw. freie Ende 7a des Ansaugrohrs 7 ragt durch die einzige Öffnung 15 bzw. Durchgangsbohrung 15a in der Wand 2a des Versorgungsrohrs 2 in das Versorgungsrohr 2. Insbesondere erstreckt sich das freie Ende 7a des Ansaugrohrs 7 bis zur Innenwand 2i des Versorgungsrohrs 2.

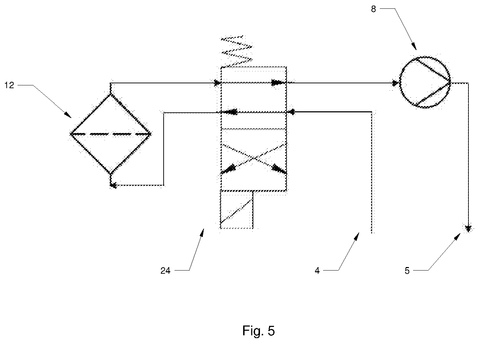

In Fig. 5 ist schematisch eine Umleiteinrichtung 24 der Vorrichtung 1 dargestellt, welche Umleiteinrichtung 24 zum Umkehren der Strömungsrichtung der abgezweigten Flüssigkeit Fa durch das Sedimentfilter 12 eingerichtet ist. Im in Fig. 5 dargestellten Beispiel ist die Umleiteinrichtung 24 ein Wegeventil oder sie weist ein solches auf. Die Umleiteinrichtung 24 bzw. das Wegeventil leitet den abgezweigten Flüssigkeitsstrom S wahlweise in einer von zwei entgegengesetzten Richtungen durch das Sedimentfilter 12. Auf diese Weise kann durch Umkehren der Strömungsrichtung der abgezweigten Flüssigkeit Fa durch das Sedimentfilter 12 das Sedimentfilter 12 bedarfsweise gereinigt werden. Im in Fig. 5 dargestellten Beispiel ist die Flüssigkeitspumpe 8 zweckmäßiger Weise zum Pumpen der Flüssigkeit F, Fa in einer einzigen Richtung, d.h. von der Einlassöffnung 4 zur Auslassöffnung 5 der Durchflusszelle 3, ausgebildet.

## Patentansprüche

1. Vorrichtung (1) zur Erfassung der Qualität einer Flüssigkeit (F) in einem Versorgungsrohr (2), insbesondere zur Erfassung der Wasserqualität in einem Wasserrohr, mit einer Durchflusszelle (3), welche eine Einlassöffnung (4), eine Auslassöffnung (5) und zumindest eine Aufnahmevorrichtung (10) zur Anordnung zumindest eines Sensors (6) aufweist, wobei die Einlassöffnung (4) und die Auslassöffnung (5) an einer zur Verbindung mit dem Versorgungsrohr (2) vorgesehenen Basisfläche (3a) der Durchflusszelle (3) vorgesehen sind, ein Ansaugrohr (7) vorgesehen ist, die Einlassöffnung (4) der Durchflusszelle (3) mit dem Ansaugrohr (7) verbunden ist, dessen freies Ende (7a) zur Anordnung im Versorgungsrohr (2) vorgesehen ist, und eine Flüssigkeitspumpe (8), zum Pumpen eines aus der Flüssigkeit (F) im Versorgungsrohr (2) abgezweigten Stroms (S) durch das Ansaugrohr (7), die Einlassöffnung (4), die Durchflusszelle (3) und durch die Auslassöffnung (5), vorgesehen und mit dem Ansaugrohr (7) verbunden ist und, dass zumindest ein Sensor (6) in der Durchflusszelle (3) angeordnet ist und durch eine Spektrometersonde, eine ionenselektive Sonde, einen elektrochemischen Sensor oder einen optischen Sensor gebildet ist, **dadurch gekennzeichnet, dass** das Ansaugrohr in seiner Längsrichtung (L) verschiebbar in der Durchflusszelle aufgenommen oder in seiner Länge verstellbar ausgebildet ist.

2. Vorrichtung (1) nach Anspruch 1, **dadurch gekennzeichnet, dass** sich in einem mit dem Versorgungsrohr (2) verbundenen Betriebszustand der Durchflusszelle (3) das freie Ende (7a) des Ansaugrohrs (7) bis zu einer Wand (2a) des Versorgungsrohrs (2) erstreckt.

3. Vorrichtung (1) nach Anspruch 1 oder 2, **dadurch gekennzeichnet, dass** die Flüssigkeitspumpe (8) an der Durchflusszelle (3) angebracht ist.

4. Vorrichtung (1) nach einem der Ansprüche 1 bis 3, **dadurch gekennzeichnet, dass** zumindest eine Verschlusseinrichtung (9) zum Absperren des Stroms (S) der Flüssigkeit (F) im Versorgungsrohr (2) in Richtung zur Durchflusszelle (3) bzw. von der Durchflusszelle (3) weg vorgesehen ist.

5. Vorrichtung (1) nach einem der Ansprüche 1 bis 4, **dadurch gekennzeichnet, dass** eine Verstelleinrichtung (11) für das Ansaugrohr (7) zum Verstellen des Ansaugrohrs (7) in Längsrichtung (L) des Ansaugrohrs (7) zwischen einer eingezogenen, im Betriebszustand der Durchflusszelle (3) vom Versorgungsrohr (2) entfernten Position und einer ausgefahrenen, im Betriebszustand der Durchflusszelle (3) zum Versorgungsrohr (2) erstreckten Position, vorgesehen ist.

6. Vorrichtung (1) nach einem der Ansprüche 1 bis 5, **dadurch gekennzeichnet, dass** in der Durchflusszelle (3), in Strömungsrichtung (R) der Flüssigkeit (F) vor der zumindest einen Aufnahmevorrichtung (10), oder im Ansaugrohr (7) ein Sedimentfilter (12) vorgesehen ist.

7. Vorrichtung (1) nach Anspruch 6, **dadurch gekennzeichnet, dass** die Flüssigkeitspumpe (8) zum Pumpen der Flüssigkeit (F) in entgegengesetzte Richtungen ausgebildet ist oder eine Umleiteinrichtung (24) zum Umkehren der Strömungsrichtung (R) der Flüssigkeit (F) durch das Sedimentfilter (12) vorgesehen ist.

8. Vorrichtung (1) nach einem der Ansprüche 1 bis 7, **dadurch gekennzeichnet, dass** zumindest ein Sensor (6) in der Durchflusszelle (3) durch einen Durchflusssensor oder einen Drucksensor gebildet ist.

9. Vorrichtung (1) nach einem der Ansprüche 1 bis 8, **dadurch gekennzeichnet, dass** zumindest eine Aufnahmevorrichtung (10) zum Einschrauben oder Einstecken zumindest eines Sensors (6) in die Durchflusszelle (3) ausgebildet ist.

10. Vorrichtung (1) nach einem der Ansprüche 1 bis 9, **dadurch gekennzeichnet, dass** die Durchflusszelle (3) und/oder das Ansaugrohr (7) zumindest teilweise aus Metall und/oder Kunststoff, vorzugsweise Polyoxymethylen, gebildet ist.

11. Vorrichtung (1) nach einem der Ansprüche 1 bis 10, **dadurch gekennzeichnet, dass** die Durchflusszelle (3) eine Entlüftungsvorrichtung (13), insbesondere ein Entlüftungsventil (13a), aufweist oder damit verbunden ist.

12. Vorrichtung (1) nach einem der Ansprüche 1 bis 11, **dadurch gekennzeichnet, dass** die Durchflusszelle (3) eine Entnahmevorrichtung (14), vorzugsweise einen Kugelhahn (14a), zur Entnahme einer Flüssigkeitsprobe aufweist.

13. Kombination eines Versorgungsrohrs (2), insbesondere Wasserrohrs, mit einer Vorrichtung (1) nach einem der Ansprüche 1 bis 12, **dadurch gekennzeichnet, dass** das Ansaugrohr (7) der Vorrichtung (1) durch eine einzige Öffnung (15) in einer Wand (2a) des Versorgungsrohrs (2) in das Versorgungsrohr (2) ragt.

14. Verfahren zum Verbinden der Vorrichtung (1) nach einem der Ansprüche 1 bis 12 mit einem Versorgungsrohr (2), insbesondere einem Wasserrohr, **dadurch gekennzeichnet, dass** eine Durchgangsbohrung (15a) in eine Wand (2a) des Versorgungsrohrs (2) eingebracht wird und an der Wand (2a), mit der Durchgangsbohrung (15a) fluchtend, eine Verschlusseinrichtung (9) zum Absperren des Stroms (S) der Flüssigkeit (F) im Versorgungsrohr (2) in Richtung zur Durchflusszelle (3) bzw. von der Durchflusszelle (3) weg, und darauf folgend die Durchflusszelle (3) fixiert werden.

15. Verfahren nach Anspruch 14, **dadurch gekennzeichnet, dass** vorzugsweise nach dem Fixieren der Durchflusszelle (3) am Versorgungsrohr (2) das Ansaugrohr (7) mittels einer Verstelleinrichtung (11) für das Ansaugrohr (7) bis zum Versorgungsrohr (2) aus der Durchflusszelle (3) ausgefahren wird, welche Verstelleinrichtung (11) zum Verstellen des Ansaugrohrs (7) in Längsrichtung (L) des Ansaugrohrs (7) zwischen einer eingezogenen, im Betriebszustand der Durchflusszelle (3) vom Versorgungsrohr (2) entfernten Position und einer ausgefahrenen, im Betriebszustand der Durchflusszelle (3) zum Versorgungsrohr (2) erstreckten Position, vorgesehen ist.

16. Verfahren nach Anspruch 15, **dadurch gekennzeichnet, dass** vor dem Ausfahren des Ansaugrohrs (7) aus der am Versorgungsrohr (2) fixierten Durchflusszelle (3) die Verschlusseinrichtung (11) geöffnet wird.

## Claims

1. A device (1) for detecting the quality of a liquid (F) in a supply pipe (2), in particular for detecting the water quality in a water pipe, comprising a flow cell (3), which comprises an inlet opening (4), an outlet opening (5) and at least one receiving device (10) for the arrangement of at least one sensor (6), wherein the inlet opening (4) and the outlet opening (5) are provided on a base surface (3a) of the flow cell (3), which base surface (3a) is intended for connection to the supply pipe (2), an intake pipe (7) is provided, the inlet opening (4) of the flow cell (3) is connected to the intake pipe (7), the free end (7a) of which is intended for arrangement in the supply pipe (2), and a liquid pump (8), for pumping a flow (S) branched off from the liquid (F) in the supply pipe (2) through the intake pipe (7), the inlet opening (4), the flow cell (3), and through the outlet opening (5), is provided and is connected to the intake pipe (7), and wherein at least one sensor (6) is arranged in the flow cell (3) and is formed by a spectrometer probe, an ion-selective probe, an electrochemical sensor, or an optical sensor, **characterised in that** said intake pipe (7) being received displaceably in its longitudinal direction (L) in the flow cell (3) or is designed to be adjustable in its length.

2. The device (1) according to claim 1, **characterised in that** the free end (7a) of the intake pipe (7) extends as far as a wall (2a) of the supply pipe (2) in an operating state of the flow cell (3) connected to the supply pipe (2).

3. The device (1) according to claim 1 or 2, **characterised in that** the liquid pump (8) is mounted on the flow cell (3).

4. The device (1) according to any one of claims 1 to 3, **characterised in that** at least one closure means (9) is provided for shutting off the flow (S) of the liquid (F) in the supply pipe (2) in the direction towards the flow cell (3) or away from the flow cell (3).

5. The device (1) according to any one of claims 1 to 4, **characterised in that** an adjustment means (11) for the intake pipe (7) is provided, for adjusting the intake pipe (7) in the longitudinal direction (L) of the intake pipe (7) between a retracted position distanced from the supply pipe (2) in the operating state of the flow cell (3) and a deployed position extended to the supply pipe (2) in the operating state of the flow cell (3).

6. The device (1) according to any one of claims 1 to 5, **characterised in that** a sediment filter (12) is provided in the flow cell (3) before the at least one receiving device (10) in the flow direction (R) of the liquid (F), or is provided in the intake pipe (7) .

7. The device (1) according to claim 6, **characterised in that** the liquid pump (8) is designed to pump the liquid (F) in opposite directions, or a rerouting means (24) for reversing the flow direction (R) of the liquid (F) through the sediment filter (12) is provided.

8. The device (1) according to any one of claims 1 to 7, **characterised in that** at least one sensor (6) in the flow cell (3) is formed by a flow sensor or a pressure sensor.

9. The device (1) according to any one of claims 1 to 8, **characterised in that** at least one receiving device (10) is designed to screw or plug at least one sensor (6) into the flow cell (3).

10. The device (1) according to any one of claims 1 to 9, **characterised in that** the flow cell (3) and/or the intake pipe (7) is formed at least in part of metal and/or plastic, preferably polyoxymethylene.

11. The device (1) according to any one of claims 1 to 10, **characterised in that** the flow cell (3) comprises or is connected to a ventilation device (13), in particular a ventilation valve (13a) .

12. The device (1) according to any one of claims 1 to 11, **characterised in that** the flow cell (3) comprises a removal device (14), preferably a ball valve (14a), for taking a liquid sample.

13. A combination of a supply pipe (2), in particular a water pipe, with a device (1) according to any one of claims 1 to 12, **characterised in that** the intake pipe (7) of the device (1) protrudes through a single opening (15) in a wall (2a) of the supply pipe (2) into the supply pipe (2).

14. A method for connecting the device (1) according to any one of claims 1 to 12 to a supply pipe (2), in particular a water pipe, **characterised in that** a through-bore (15a) is formed in a wall (2a) of the supply pipe (2), and a closure means (9) for shutting off the flow (S) of the liquid (F) in the supply pipe (2) in the direction towards the flow cell (3) or away from the flow cell (3), and then the flow cell (3) are fixed to the wall (2a), in alignment with the through-bore (15a).

15. The method according to claim 14, **characterised in that** the intake pipe (7) is deployed from the flow cell (3) by means of an adjustment means (11) for the intake pipe (7), as far as the supply pipe (2), preferably once the flow cell (3) has been fixed to the supply pipe (2), which adjustment means (11) is intended to adjust the intake pipe (7) in the longitudinal direction (L) of the intake pipe (7) between a retracted position, distanced from the supply pipe (2) in the operating state of the flow cell (3) and a deployed position, extended to the supply pipe (2) in the operating state of the flow cell (3).

16. The method according to claim 15, **characterised in that** the closure means (11) is opened before the intake pipe (7) is deployed from the flow cell (3) fixed the supply pipe (2).

## Revendications

1. Dispositif (1) pour détecter la qualité d'un liquide (F) dans une conduite d'alimentation (2), en particulier pour détecter la qualité de l'eau dans une conduite d'eau, avec une cellule à circulation (3), qui comporte une ouverture d'entrée (4), une ouverture de sortie (5) et au moins un dispositif de réception (10) pour l'agencement d'au moins un capteur (6), où l'ouverture d'entrée (4) et l'ouverture de sortie (5) sont prévues sur une surface de base (3a) de la cellule à circulation (3) prévue pour la liaison avec la conduite d'alimentation (2), une conduite d'aspiration (7) est prévue, l'ouverture d'entrée (4) de la cellule à circulation (3) est reliée à la conduite d'aspiration (7), dont l'extrémité libre (7a) est prévue pour l'agencement dans la conduite d'alimentation (2), et une pompe à liquide (8), pour pomper un courant (S) dérivé du liquide (F) dans la conduite d'alimentation (2) à travers la conduite d'aspiration (7), l'ouverture d'entrée (4), la cellule à circulation (3) et à travers l'ouverture de sortie (5), est prévue et reliée à la conduite d'aspiration (7), et en ce qu'au moins un capteur (6) est agencé dans la cellule à circulation (3) et est formé par une sonde de spectromètre, une sonde sélective pour les ions, un capteur électrochimique ou un capteur optique, **caractérisé en ce que** la conduite d'aspiration est reçue dans la cellule à circulation de manière mobile dans sa direction longitudinale (L) ou conçue pour être déplaçable dans sa longueur.

2. Dispositif (1) selon la revendication 1, **caractérisé en ce que** dans un état de fonctionnement de la cellule à circulation (3) relié à la conduite d'alimentation (2), l'extrémité libre (7a) de la conduite d'aspiration (7) s'étend jusqu'à une paroi (2a) de la conduite d'alimentation (2).

3. Dispositif (1) selon la revendication 1 ou 2, **caractérisé en ce que** la pompe à liquide (8) est fixée à la cellule à circulation (3).

4. Dispositif (1) selon l'une des revendications 1 à 3, **caractérisé en ce qu'**au moins un dispositif d'obturation (9) pour couper le courant (S) du liquide (F) dans la conduite d'alimentation (2) en direction de la cellule à circulation (3) ou depuis la cellule à circulation (3) est prévu.

5. Dispositif (1) selon l'une des revendications 1 à 4, **caractérisé en ce qu'**un dispositif de déplacement (11) pour la conduite d'aspiration (7) est prévu pour déplacer la conduite d'aspiration (7) dans la direction longitudinale (L) de la conduite d'aspiration (7) entre une position rentrée, éloignée de la conduite d'alimentation (2) dans l'état de fonctionnement de la cellule à circulation (3), et une position sortie, qui s'étend jusqu'à la conduite d'alimentation (2) dans l'état de fonctionnement de la cellule à circulation (3).

6. Dispositif (1) selon l'une des revendications 1 à 5, **caractérisé en ce qu'**un filtre à sédiments (12) est prévu dans la cellule à circulation (3), dans la direction d'écoulement (R) du liquide (F) devant le au moins un dispositif de réception (10), ou dans la conduite d'aspiration (7).

7. Dispositif (1) selon la revendication 6, **caractérisé en ce que** la pompe à liquide (8) est conçue pour pomper le liquide (F) dans des directions opposées ou un dispositif de dérivation (24) pour inverser la direction d'écoulement (R) du liquide (F) à travers le filtre à sédiments (12) est prévu.

8. Dispositif (1) selon l'une des revendications 1 à 7, **caractérisé en ce qu'**au moins un capteur (6) est formé dans la cellule à circulation (3) par un capteur de circulation ou un capteur de pression.

9. Dispositif (1) selon l'une des revendications 1 à 8, **caractérisé en ce qu'**au moins un dispositif de réception (10) est conçu pour visser ou enficher au moins un capteur (6) dans la cellule à circulation (3).

10. Dispositif (1) selon l'une des revendications 1 à 9, **caractérisé en ce que** la cellule à circulation (3) et/ou la conduite d'aspiration (7) est formée au moins partiellement en métal et/ou en matière synthétique, de préférence en polyoxyméthylène.

11. Dispositif (1) selon l'une des revendications 1 à 10, **caractérisé en ce que** la cellule à circulation (3) comporte un dispositif de purge d'air (13), en particulier une vanne de purge d'air (13a), ou est reliée à celui-ci.

12. Dispositif (1) selon l'une des revendications 1 à 11, **caractérisé en ce que** la cellule à circulation (3) comporte un dispositif de prélèvement (14), de préférence un robinet à boisseau sphérique (14a), pour prélever un échantillon de liquide.

13. Combinaison d'une conduite d'alimentation (2), en particulier une conduite d'eau, avec un dispositif (1) selon l'une des revendications 1 à 12, **caractérisée en ce que** la conduite d'aspiration (7) du dispositif (1) fait saillie dans la conduite d'alimentation (2) à travers une seule ouverture (15) dans une paroi (2a) de la conduite d'alimentation (2).

14. Procédé pour relier le dispositif (1) selon l'une des revendications 1 à 12 avec une conduite d'alimentation (2), en particulier une conduite d'eau, **caractérisé en ce qu'**un trou traversant (15a) est pratiqué dans une paroi (2a) de la conduite d'alimentation (2) et sur la paroi (2a), en alignement avec le trou traversant (15a), un dispositif d'obturation (9) pour couper le courant (S) du liquide (F) dans la conduite d'alimentation (2) en direction de la cellule à circulation (3) ou depuis la cellule à circulation (3), puis la cellule à circulation (3) sont fixés.

15. Procédé selon la revendication 14, **caractérisé en ce que**, de préférence après la fixation de la cellule à circulation (3) sur la conduite d'alimentation (2), la conduite d'aspiration (7) est retirée de la cellule à circulation (3) jusqu'à la conduite d'alimentation (2) au moyen d'un dispositif de déplacement (11) pour la conduite d'aspiration (7), lequel dispositif de déplacement (11) est prévu pour le déplacement de la conduite d'aspiration (7) dans la direction longitudinale (L) de la conduite d'aspiration (7) entre une position rentrée, éloignée de la conduite d'alimentation (2) dans l'état de fonctionnement de la cellule à circulation (3) et une position sortie, qui s'étend jusqu'à la conduite d'alimentation (2) dans l'état de fonctionnement de la cellule à circulation (3).

16. Procédé selon la revendication 15, **caractérisé en ce que** le dispositif d'obturation (11) est ouvert avant la sortie de la conduite d'aspiration (7) hors de la cellule à circulation (3) fixée sur la conduite d'alimentation (2).
